(19) Europäisches Patentamt
European Patent Office
Office européen des brevets

(11) Veröffentlichungsnummer: **0 298 271 B1**

# (12) EUROPÄISCHE PATENTSCHRIFT

(45) Veröffentlichungstag der Patentschrift: **08.01.92**

(51) Int. Cl.5: **A61K 7/043**, A61K 31/415, A61K 31/325, A61K 31/13

(21) Anmeldenummer: **88109260.5**

(22) Anmeldetag: **10.06.88**

## (54) Antimykotisch wirksamer Nagellack sowie Verfahren zu dessen Herstellung.

(30) Priorität: **16.06.87 DE 3720147**

(43) Veröffentlichungstag der Anmeldung:
**11.01.89 Patentblatt 89/02**

(45) Bekanntmachung des Hinweises auf die Patenterteilung:
**08.01.92 Patentblatt 92/02**

(84) Benannte Vertragsstaaten:
**AT BE CH DE FR GB IT LI LU NL SE**

(56) Entgegenhaltungen:
**EP-A- 0 055 397**
**WO-A-87/02580**

(73) Patentinhaber: **HOECHST AKTIENGESELLSCHAFT**
**Postfach 80 03 20**
**W-6230 Frankfurt am Main 80(DE)**

(72) Erfinder: **Bohn, Manfred, Dr.**
**Schweriner Weg 10**
**W-6238 Hofheim am Taunus(DE)**
Erfinder: **Dittmar, Walter, Dr.**
**Uhlandstr. 10**
**W-6238 Hofheim am Taunus(DE)**
Erfinder: **Kraemer, Karl, Dr.**
**Im Buchenhain 37**
**W-6070 Langen(DE)**
Erfinder: **Peil, Heinz Georg, Dr.**
**Homburger Str. 52**
**W-6350 Bad Nauheim(DE)**
Erfinder: **Futterer, Eberhard, Dr.**
**Im Stückes 50**
**W-6233 Kelkheim (Taunus)(DE)**

EP 0 298 271 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Pilzerkrankungen der Nägel (Onychomykosen) sind hartnäckige Krankheitsformen, die bisher nicht befriedigend behandelt werden konnten. Unter dem Begriff Onychomykosen werden verschiedene Typen von Nagelmykosen zusammengefaßt, von denen die durch Dermatophyten verursachten am schwierigsten zu behandeln sind, während die durch Hefepilze verursachten Nagelmykosen bisher am ehesten erfolgreich therapiert werden konnten.

Die Tücke der durch Dermatophyten bedingten Onychomykosen besteht auch darin, daß sie erheblich zur Verbreitung infektöser Pilze beitragen. Zu ihrer Behandlung hat man bisher, allerdings ohne durchschlagenen Erfolg, verschiedene Wege beschritten.

Eine Behandlungsmethode, die systemische, bestand darin, daß man pilzhemmende Mittel oral verabreichte. Dies erforderte eine Langzeitbehandlung, die erfahrungsgemäß zu Intoxikationen führen kann.

Eine andere Methode besteht darin, die Nägel chirurgisch oder durch Einwirkung von Chemikalien zu entfernen und zu hoffen, daß gesunde, nicht befallene Nägel nachwachsen. Diese Methode ist naturgemäß sehr aggressiv und gibt außerdem auch keine Gewähr, daß die Nägel in der natürlichen Form nachwachsen; vielmehr sind die nachwachsenden Nägel häufig verunstaltet.

Eine dritte, aber schonende Methode besteht darin, daß man die Nägel lokal mit spezifischen, antimykotische wirksamen Stoffen behandelt. Hierbei hat man die unterschiedlichsten Behandlungsmethoden versucht. So hat man in einer kombinierten Behandlung die Nägel zunächst mit Lösungen der antimykotisch wirksamen Stoffe behandelt und nachts jeweils Cremeverbände angelegt. Auch diese Behandlungsmethode ist für den Patienten naturgemäß sehr unerfreulich und psychisch belastend. Einmal ist die Behandlung der Nägel mit Lösungen mehrmals täglich erforderlich. Zum anderen müssen sie vor allem nachts mit Verbänden versehen werden. Ferner ist ein ständiges Abfeilen der erkrankten Nägel notwendig, was sowohl mühsam ist als auch zur Verbreitung der Erreger beiträgt. Dies alles führt dazu, daß die gewöhnlich vielmonatige Behandlung von den Patienten vielfach nicht durchgehalten wird, sie vielmehr entmutigt und nachlässig werden und daß damit der Therapieerfolg ausbleibt. Der Behandlungserfolg wird bei dieser Methode weiterhin dadurch beeinträchtigt, daß die Lösungen und Cremes gewöhnlich mit Wasser mischbar bzw. hydrophil sind und daher beim Waschen, Baden und Duschen von der Nageloberfläche wieder entfernt bzw. aus dem Nagel herausgelöst werden können, mithin also danach wieder neu aufgetragen werden müssen.

Man hat daher große Hoffnungen in eine völlig andere Methode gesetzt, nämlich in die Behandlung mit einem Nagellack, der den antimykotisch wirksamen Stoff Sulbentin und als Filmbildner Nitrocellulose enthält;Sulbentin ist eine Thiadiazinverbindung der Formel

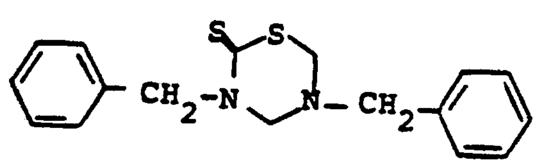

Obwohl diese Methode bereits seit etwa 20 Jahren praktiziert wird, hat sie keinen allgemeinen Eingang in die Therapie gefunden, da mit diesen Nagellacken praktisch nur leichtere Nagelmykosen bekämpft werden können. Ein zufriedenstellender Erfolg blieb dieser Zubereitung vermutlich auch mangels ausreichender Bioverfügbarkeit des Wirkstoffs aus dem nach dem Trocknen des Lackes vorliegenden Feststoffsystem versagt.

Daher hat man viele Fälle, insbesondere die schwereren, nach wie vor mit den oben beschriebenen chirurgischen oder chemischen Methoden bzw. mit der kombinierten Lösungs- und Creme-Therapie behandelt.

Ein entscheidender Fortschritt auf dem Gebiet der Behandlung bzw. Verhinderung von Nagelmykosen wurde durch das Auftragen des in der Patentanmeldung DE-A-35 44 983.7 (Stand der Technik nach Artikel 54(3) EPÜ) vorgeschlagenen Nagellacks auf die Nägel, insbesondere auf die erkrankten Nägel, erzielt; dieser Nagellack zeichnet sich durch einen Gehalt an einem wasserunlöslichen Filmbildner und mindestens ein 1-Hydroxy-2-pyridon (als antimykotisch wirksamen Stoff) der folgenden Formel aus:

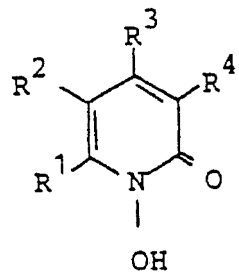

In der Formel bedeuten
$R^1$ einen "gesättigten" Kohlenwasserstoff-Rest mit 6 bis 9, vorzugsweise 6 bis 8 C-Atomen,
einer der Reste $R^2$ und $R^4$ ein H-Atom und der andere H, $CH_3$ oder $C_2H_5$ und
$R^3$ einen Alkylrest mit 1 oder 2 C-Atomen.

Der Begriff "gesättigt" bezeichnet hierbei solche Reste, die keine ethylenischen oder acetylenischen Bindungen enthalten.

Die Wirkstoffe können sowohl in freier Form als auch in Form ihrer Salze zugegen sein.

Mit diesem Nagellack läßt sich bei der Behandlung von Nagelmykosen eine durchgreifende Heilung erzielen, wobei der Nagel gewöhnlich ohne Deformierung nachwächst. Im Hinblick auf die vorherigen schlechten Therapieerfahrungen ist dies ein überaus wichtiger Befund.

Der Nagellack eignet sich auch zur prophylaktischen Anwendung gegen Nagelmykosen, wobei ein ausreichend hohes Wirkstoffdepot im Nagel erreicht wird, so daß es im Falle einer Pilzkontamination nicht zum Ausbruch einer durch Pilze hervorgerufenen Nagelerkrankung kommt.

In weiterer Bearbeitung dieses Sachgebietes wurde nun gefunden, daß sich ähnliche Vorteile auch durch den Einsatz eines Nagellacks mit einem Gehalt an mindestens einem speziellen wasserunlöslichen Filmbildner und mindestens einem speziellen Imidazol-, Thiacarbaminsäure- oder Propenylnaphthalinmethanamin-Derivat als antimykotisch wirksamen Stoff erzielen lassen.

Erfindungsgegenstand ist daher ein antimykotisch wirksamer Nagellack, enthaltend mindestens eine wasserunlöslichen Filmbildner und mindestens einen antimykotisch wirksamen Stoff; der Nagellack ist dadurch gekennzeichnet,
daß die wasserunlöslichen Filmbildner ausgewählt sind aus der Gruppe
Polyvinylacetat,
partiell verseiftes Polyvinylacetat,
Mischpolymerisate aus Vinylacetat einerseits und Acrylsäure oder Crotonsäure oder Maleinsäuremonoalkylester andererseits,
ternäre Mischpolymerisate aus Vinylacetat einerseits und Crotonsäure und Vinylneodecanoat andererseits,
ternäre Mischpolymerisate aus Vinylacetat einerseits und Crotonsäure sowie Vinylpropionat andererseits,
Mischpolymerisate aus Methylvinylether und Maleinsäuremonoalkylester,
Mischpolymerisate aus Fettsäurevinylester und Acrylsäure oder Methacrylsäure,
Mischpolymerisate aus N-Vinylpyrrolidon, Methacrylsäure und Methacrylsäurealkylester,
Mischpolymerisate aus Acrylsäure und Methacrylsäure oder Acrylsäurealkylester oder Methacrylsäurealkylester,
Polyvinylacetale,
Polyvinylbutyrale,
alkylsubstituierte Poly-N-vinylpyrrolidone, und
Alkylester von Mischpolymerisaten aus Olefinen und Maleinsäureanhydrid,
und daß die antimykotisch wirksamen Stoffe aus der folgenden Gruppe ausgewählt sind:

**Tioconazol (ein Imidazolderivat):** 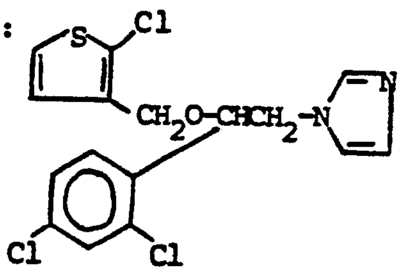

**Econazol ( " " ):** 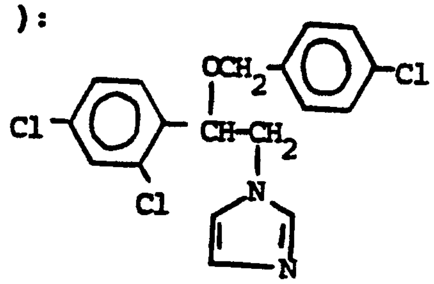

**Oxiconazol ( " " ):** 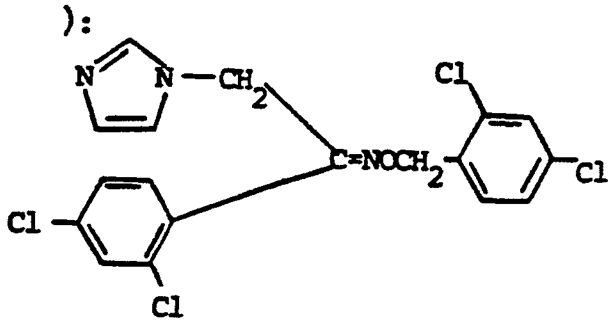

**Miconazol (ein Imidazolderivat):**

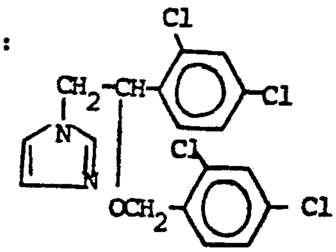

**Tolnaftat (ein Thiocarbaminsäurederivat):**

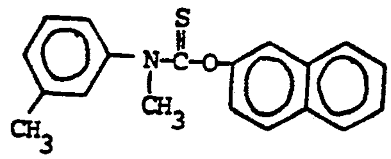

**Naftifinhydrochlorid (ein Propenylnaphthalinmethanaminderivat):**

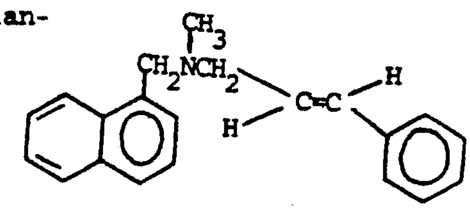

· HCl

Die wasserunlöslichen Filmbildner sind weitgehend identisch mit den auch in der vorerwähnten Patentanmeldung genannten Filmbildnern. In den Estern sind die Alkylreste gewöhnlich kurzkettig und haben meistens nicht mehr als 4 C-Atome. Wenn die Filmbildner die Einheiten von Methacrylsäure oder Methacrylsäurederivaten enthalten, sollen diese vorteilhaft nicht mehr als etwa 15 Mol.-% des Gesamtpolymeren betragen.

Die vorerwähnten wasserunlöslichen Filmbildner können gegebenenfalls auch mit Cellulosenitrat vermischt werden bzw. sein; das Cellulosenitrat soll dann jedoch nicht mehr als höchstens etwa 50 Gew.-% der gesamten Filmbildner-Komponente ausmachen.

Ein bevorzugter wasserunlöslicher Filmbildner ist das Mischpolymerisat aus Methylvinylether und Maleinsäuremonoalkylester - vorzugsweise Maleinsäuremono-n-butylester. In diesem Mischpolymerisat sind die Einheiten der beiden Komponenten vorzugsweise etwa im gleichen Verhältnis enthalten.

Die in dem erfindungsgemäßen Nagellack vorhandenen antimykotischen Stoffe sind bekannte Verbindungen, deren antimykotische Eigenschaften ebenfalls bekannt sind. Bevorzugte antimykotische Stoffe sind hier Tioconazol und/oder Econazol. Die Stoffe können sowohl einzeln als auch in Mischung miteinander angewandt werden; die Salze sind ebenso einsetzbar.

Der Gehalt an Wirkstoff in dem erfindungsgemäßen Nagellack ist von der Struktur eines jeden Wirkstoffs und damit von dessen Freigabe aus dem Lackfilm, seinem Penetrationsverhalten im Nagel und seinen antimikrobiellen Eigenschaften abhängig.

Der erfindungsgemäße Nagellack wird vorzugsweise als medizinischer Nagellack angewandt, der eine wirksame Menge des antimykotischen Wirkstoffs enthält, die zur Abtötung der Nagelmykosen verursachenden Dermatophyten ausreicht.

In dem Nagellack- also der Lösemittel enthaltenden Anwendungsform - ist der Wirkstoff im allgemeinen in einer Menge von etwa 0,5 bis 20, vorzugsweise von etwa 2 bis 15 Gew.-% enthalten.

Jeweils bezogen auf die Menge der nichtflüchtigen Bestandteile - d.i. die Summe der Filmbildner, der gegebenenfalls vorhandenen Pigmente, Weichmacher und anderen nichtflüchtigen Zusätzen sowie Wirkstoff - ist in den erfindungsgemäßen Nagellacken der Wirkstoff im allgemeinen in einer Menge von etwa 2 bis

80, vorzugsweise von etwa 10 bis 60 und insbesondere von etwa 20 bis 40 Gew.-% enthalten.

Als Lösemittel für den erfindungsgemäßen Nagellack kommen Stoffe wie in Kosmetika übliche Kohlenwasserstoffe, halogenierte Kohlenwasserstoffe, Alkohole, Ether, Ketone und Ester in Betracht, insbesondere Essigsäureester von einwertigen Alkoholen (Essigsäureethylester, Essigsäure-n-butylester, etc.), gegebenenfalls im Gemisch mit aromatischen Kohlenwasserstoffen wie Toluol und/oder Alkoholen wie Ethanol oder Isopropanol und/oder aliphatischen Sulfoxiden und Sulfonen wie etwa Dimethylsulfoxid oder Sulfolan in Frage.

Die Kombination der Lösemittel ist bekanntlich von entscheidender Bedeutung für die Trockenzeit, Verstreichbarkeit und andere wichtige Eigenschaften des Lackes bzw. des Lackfilms. Das Lösemittelsystem besteht vorzugsweise aus einer optimalen Mischung aus Niedrigsiedern (= Lösemittel mit einem Siedepunkt bis etwa 100 °C) und Mittelsiedern (= Lösemittel mit einem Siedepunkt bis etwa 150 °C), gegebenenfalls mit einem kleinen Anteil Hochsiedern (= Lösemittel mit einem Siedepunkt über~150 °C)>

Die erfindungsgemäßen Nagellacke können weiterhin in Kosmetika gebräuchliche Zusätze enthalten, wie Weiohmacher auf Phthalat-oder Campherbasis, Farbstoffe bzw. Farbpigmente, Perlglanzmittel, Sedimentationsverzögerer, Sulfonamidharze, Silikate, Riechstoffe, Netzmittel wie Natriumdioctysulfosuccinat, Lanolinderivate, Lichtschutzmittel wie 2-Hydroxy-4-methoxybenzophenon, antibakteriell wirksame Substanzen und Stoffe mit keratolytischer und/oder keratoplastischer Wirkung, wie Ammoniumsulfit, Ester und Salze der Thioglykolsäure, Harnstoff, Allantoin, Enzyme und Salicylsäure.

Gefärbte bzw. pigmentierte Nagellacke haben z.B. den Vorteil, daß die erfindungsgemäße Zubereitung dem Schönheitsempfinden des Patienten angepaßt werden kann.

Die Herstellung des Nagellackes erfolgt in üblicher Weise durch Zusammengeben der einzelnen Komponenten und eine - soweit erforderlich - der jeweiligen Zubereitung angepaßte Weiterverarbeitung.

Die erfindungsgemäßen Nagellacke unterscheiden sich auch grundsätzlich von den aus der europäischen Patentschrift 55 397 bekannten antimykotischen Mittel, die als Wirkstoffe Azolderivate, insbesondere Imidazol- und Triazol-Derivate, enthalten. Diese antimykotischen Mittel sollen als wasserlöslicher Film aufgetragen werden, eine Depotwirkung aufweisen und eine Kurzzeittherapie ermöglichen. Sie sollen sich auch zur Behandlung von Nagelmykosen eignen und sowohl in Lösungen als auch in Sprays, die nach dem Trocknen einen wasserlöslichen Film bilden, eingesetzt werden. Die Verwendung derartiger wasserlöslicher Bindemittel bewirkt naturgemäß, daß das aufgetragene Mittel bei jedem Waschen mehr oder weniger entfernt wird.

Die Verwendung wasserunlöslicher filmbildender Polymerer gemäß der vorliegenden Erfindung steht in krassem Gegensatz zu der Aussage in der EP-B 55 397, wonach dann, wenn man anstelle der dort beschriebenen Formulierungen mit wasserlöslichen Polymeren wasserunlösliche Polymere, z.B. "Methacrylate" verwendet, die Mykose verschlimmert wird. Der bekannte Sulbentin-haltige Nagellack enthält zwar mit der Nitrocellulose einen wasserunlöslichen Filmbildner; der Nagellack hat sich aber - wie eingangs erwähnt - wegen seiner mangelhaften antimykotischen Wirksamkeit nicht durchsetzen können. Mit Hilfe der erfindungsgemäßen Nagellacke, die nach dem Trocknen wasserunlöslich werdende filmbildende Polymere enthalten, läßt sich die Nagelmykose dagegen erfolgreich behandeln.

Der Wirksamkeitstest der erfindungsgemäßen Nagellacke erfolgte an verhornter exzidierter Schweinehaut. Das angewandte Prüfverfahren erlaubt es, die Freigabe der Wirkstoffe aus dem nach dem Trocknen des Nagellacks vorliegenden Feststoffsystem und ihr Penetrationsvermögen in verhorntem Gewebe zu prüfen.

Die vorliegende Erfindung wird durch die folgenden Beispiele näher erläutert; die Beispiele enthalten auch einen Vergleich mit einem Sulbentin-haltigen Nagellack. Die prozentualen Mengenangaben sind auf das Gewicht bezogen. T bedeutet Gewichtsteile.

Beispiele 1 - 6 sowie Vergleichsbeispiel (mit Sulbentinhaltigem Nagellack):

Auf die Oberfläche von geschorenen Schweinehautstücken wurden zunächst Pilze des Stammes Trichophyton mentagrophytes 109 aufgeimpft und dort angezüchtet. Nach einer Vorzuchtzeit von 3 Tagen war der Pilz in das verhornte Gewebe eingewachsen. Anschließend wurde die Hautoberfläche abgewaschen und mit 0,5 %igen Zubereitungen der Verbindungen 1 bis 6, jeweils gelöst in einem Gemisch aus 45 T Isopropanol, 29,5 T Essigsäureethylester, 10 T Dimethylsulfoxid und 15 T eines Mischpolymerisats aus gleichen Molteilen Methylvinylether und Maleinsäuremono-n-butylester bei Raumtemperatur behandelt. Nach 2 Stunden Einwirkungszeit wurde die Lackzubereitung durch Klebstreifenabrisse wieder von der Hautoberfläche entfernt. Nach mechanischer Homogenisierung des behandelten verhornten Gewebes wurde diese nach entsprechender Verdünnung zur Keimzahlbestimmung auf Agarplatten ausgestrichen.

Der gleiche Test wurde auch mit der Vergleichssubstanz Sulbentin durchgeführt.

In der nachfolgenden Tabelle sind die Ergebnisse dieser Versuche aufgeführt. Die Zahlenangaben beziehen sich dabei auf die prozentuale Abnahme der Keimeinheiten in den behandelten Hautstücken im Vergleich zu dem Kontrollwachstum von unbehandelter Haut.

**Tabelle**

**Fungizide (%) an exzidierter Haut**

**Haut:** Schwein (Rücken)
**Präparate:** Nagellacke* mit 0,5 % Wirkstoff

| | | | |
|---|---|---|---|
| Beispiel: | 1 | Tioconazol | 97,1 |
| | 2 | Econazol | 92,9 |
| | 3 | Oxiconazol | 88,9 |
| | 4 | Miconazol | 85,6 |
| | 5 | Tolnaftat | 87,6 |
| | 6 | Naftifinhydrochlorid | 90,0 |
| Vergleich: | | Sulbentin | 82,9 |

* Zusammensetzung der Lackgrundlage:
45 T Isopropanol, 29,5 T Essigsäureethylester, 10 T Dimethylsulfoxid, 15 T Methylvinylether/Maleinsäure-monobutylester Mischpolymerisat.

Beispiele 7 - 9

In den nachstehenden Beispielen sind Rezepturen für die erfindungsgemäßen Nagellack-Zubereitungen angegeben. Die Nagellacke wurden durch Lösen der verschiedenen Komponenten in den Lösemitteln hergestellt.

7. Isopropylakohol 33,00 %
Essigsäureethylester 33,00 %
50 %ige Lösung eines Mischpolymerisates aus Methylvinylether und Maleinsäuremonobutylester in Isopropylalkohol 30,00 %
Tioconazol 4,00 %

8. Isopropylalkohol 55,00 %
Essigsäureethylester 32,00 %
Wasser 4,00 %
Cellulosenitrat 3,10 %
Dibutylphthalat 0,60 %
Polyvinylbutyral 3,80 %
Naftifinhydrochlorid 1,50 %

9. Isopropylalkohol 28,70 %

Essigsäureethylester 28,60 %
Wasser 10,70 %
50 %ige Lösung eines Mischpolymerisates aus Methylvinylether und Maleinsäuremonobutylester in Isopropylalkohol 30,00 %
Econazolnitrat 2,00 %

## Patentansprüche

1. Antimykotisch wirksamer Nagellack, enthaltend mindestens einen wasserunlöslichen Filmbildner und mindestens einen antimykotisch wirksamen Stoff, dadurch gekennzeichnet,
daß die wasserunlöslichen Filmbildner ausgewählt sind aus der Gruppe
Polyvinylacetat,
partiell verseiftes Polyvinylacetat,
Mischpolymerisate aus Vinylacetat einerseits und Acrylsäure oder Crotonsäure oder Maleinsäuremonoalkylester andererseits,
ternäre Mischpolymerisate aus Vinylacetat einerseits und Crotonsäure und Vinylneodecanoat andererseits,
ternäre Mischpolymerisate aus Vinylacetat einerseits und Crotonsäure sowie Vinylpropionat andererseits,
Mischpolymerisate aus Methylvinylether und Maleinsäuremonoalkylester,
Mischpolymerisate aus Fettsäurevinylester und Acrylsäure oder Methacrylsäure,
Mischpolymerisate aus N-Vinylpyrrolidon, Methacrylsäure und Methacrylsäurealkylester,
Mischpolymerisate aus Acrylsäure und Methacrylsäure oder Acrylsäurealkylester oder Methacrylsäurealkylester,
Polyvinylacetale,
Polyvinylbutyrale,
alkylsubstituierte Poly-N-vinylpyrrolidone, und Alkylester von Mischpolymerisaten aus Olefinen und Maleinsäureanhydrid,
und daß die antimykotisch wirksamen Stoffe aus der folgenden Gruppe ausgewählt sind:
Tioconazol,
Econazol,
Oxiconazol,
Miconazol,
Tolnaftat und
Naftifinhydrochlorid.

2. Nagellack nach Anspruch 1, dadurch gekennzeichnet, daß der wasserunlösliche Filmbildner ein Mischpolymerisat aus Methylvinylether und Maleinsäuremonoalkylester ist.

3. Nagellack nach Anspruch 1 oder 2, dadurch gekennzeichnet, daß der wasserunlösliche Filmbildner ein Mischpolymerisat aus Methylvinylether und Maleinsäuremono-n-butylester ist.

4. Nagellack nach einem oder mehreren der Ansprüche 1 bis 3, dadurch gekennzeichnet, daß der antimykotisch wirksame Stoff Tioconazol und/oder Econazol ist.

5. Nagellack nach einem oder mehreren der Ansprüche 1 bis 4, dadurch gekennzeichnet, daß der Wirkstoff in einer Menge von etwa 2 bis 80 Gew.-%, bezogen auf die Menge der nichtflüchtigen Bestandteile, enthalten ist.

6. Nagellack nach einem oder mehreren der Ansprüche 1 bis 5, dadurch gekennzeichnet, daß der Wirkstoff in einer Menge von etwa 10 bis 60 Gew.-%, bezogen auf die Menge der nichtflüchtigen Bestandteile, enthalten ist.

7. Nagellack nach einem oder mehreren der Ansprüche 1 bis 6, dadurch gekennzeichnet, daß er den Wirkstoff in einer Menge von etwa 0,5 bis 20 Gew.-%, bezogen auf die Menge der flüchtigen und der nichtflüchtigen Bestandteile, enthält.

8. Nagellack nach einem oder mehreren der Ansprüche 1 bis 7, dadurch gekennzeichnet, daß er den

Wirkstoff in einer Menge von etwa 2 bis 15 Gew.-%, bezogen auf die Menge der flüchtigen und der nichtflüchtigen Bestandteile, enthält.

9. Verfahren zur Herstellung eines Nagellacks mit einem Gehalt an mindestens einem wasserunlöslichen Filmbildner und mindestens einem antimykotisch wirksamen Stoff, dadurch gekennzeichnet, daß man mindestens einen wasserunlöslichen Filmbildner der in Anspruch 1 definierten Art mit mindestens einem antimykotisch wirksamen Stoff der ebenfalls in Anspruch 1 definierten Art sowie gegebenenfalls mit anderen für die Herstellung von Nagellack üblichen Komponenten vermischt.

10. Verwendung einer oder mehrerer der in Anspruch 1 definierten antimykotisch wirksamen Stoffe als Zusatzmittel in Nagellack(en) mit mindestens einem wasserunlöslichen Filmbildner.

## Claims

1. A nail lacquer having antimycotic activity, containing at least one film-former which is insoluble in water and at least one substance having antimycotic activity, comprising the features that the film-formers which are insoluble in water are selected from the group comprising
polyvinyl acetate, partially hydrolyzed polyvinyl acetate, copolymers of vinyl acetate on the one hand and acrylic acid or crotonic acid or monoalkyl maleates on the other hand, ternary copolymers of vinyl acetate on the one hand and crotonic acid and vinyl neodecanoate on the other hand, ternary copolymers of vinyl acetate on the one hand and crotonic acid and vinyl propionate on the other hand, copolymers of methyl vinyl ether and monoalkyl maleates, copolymers of fatty acid vinyl esters and acrylic acid or methacrylic acid, copolymers of N-vinylpyrrolidone, methacrylic acid and alkyl methacrylates, copolymers of acrylic acid and methacrylic acid or alkyl acrylates or alkyl methacrylates, polyvinyl acetals, polyvinyl butyrals, alkyl-substituted poly-N-vinylpyrrolidones, and alkyl esters of copolymers of olefins and maleic anhydride, and that the substances having antimycotic activity are selected from the following group: tioconazole, econazole, oxiconazole, miconazole, tolnaftate and naftifine hydrochloride.

2. A nail lacquer as claimed in claim 1, wherein the film-former which is insoluble in water is a copolymer of methyl vinyl ether and monoalkyl maleate.

3. A nail lacquer as claimed in claim 1 or 2, wherein the film-former which is insoluble in water is a copolymer of methyl vinyl ether and mono-n-butyl maleate.

4. A nail lacquer as claimed in one or more of claims 1 to 3, wherein the substance having antimycotic activity is tioconazole and/or econazole.

5. A nail lacquer as claimed in one or more of claims 1 to 4, which contains the active substance in an amount of about 2 to 80 % by weight based on the amount of non-volatile ingredients.

6. A nail lacquer as claimed in one or more of claims 1 to 5, which contains the active substance in an amount of about 10 to 60 % by weight based on the amount of non-volatile ingredients.

7. A nail lacquer as claimed in one or more of claims 1 to 6, which contains the active substance in an. amount of about 0.5 to 20 % by weight based on the amount of volatile and non-volatile ingredients.

8. A nail lacquer as claimed in one or more of claims 1 to 7, which contains the active substance in an amount of about 2 to 15 % by weight based on the amount of volatile and non-volatile ingredients.

9. A process for the preparation of a nail lacquer containing at least one film-former which is insoluble in water and at least one substance having antimycotic activity, which comprises mixing at least one film-former which is insoluble in water and is of the type defined in claim 1 with at least one substance which has antimycotic activity and is likewise of the type defined in claim 1, as well as, where appropriate, with other components customary for the preparation of nail lacquer.

10. The use of one or more of the substances which have antimycotic activity and are defined in claim 1 as additives in nail lacquer(s) containing at least one film-former which is insoluble in water.

## Revendications

1. Vernis à ongles à action antimycosique, contenant au moins un agent feuillogène insoluble dans l'eau et au moins une substance active antimycosique, caractérisé en ce que les agents feuillogènes insolubles dans l'eau sont choisis parmi
le poly(acétate de vinyle),
un poly(acétate de vinyle) partiellement saponifié,
des copolymères d'acétate de vinyle, d'une part, et d'acide acrylique ou d'acide crotonique ou d'un ester monoalkylique d'acide maléique d'autre part,
des copolymères ternaires d'acétate de vinyle d'une part, et d'acide crotonique et de néodécanoate de vinyle d'autre part,
des copolymères ternaires d'acétate de vinyle d'une part, et d'acide crotonique ainsi que de propionate de vinyle d'autre part,
des copolymères d'éther méthylvinylique et d'un ester monoalkylique d'acide maléique,
des copolymères d'ester vinylique d'acide gras et d'acide acrylique ou d'acide méthacrylique,
des copolymères de N-vinylpyrrolidone, acide méthacrylique et méthacrylate d'alkyle,
des copolymères d'acide acrylique et d'acide méthacrylique ou d'acrylate d'alkyle ou de méthacrylate d'alkyle,
des poly(acétal de vinyle)s,
des poly(butyral de vinyle)s,
des poly-N-vinylpyrrolidones substituées et des esters alkyliques de copolymères d'oléfines et d'anhydride maléique,
et en ce que les substances à activité antimycosique sont choisies parmi les suivantes:
le tioconazole,
l'éconazole,
l'oxiconazole,
le miconazole,
le tolnaftate et
le chlorhydrate de naftifine.

2. Vernis à ongles selon la revendication 1, caractérisé en ce que l'agent feuillogène insoluble dans l'eau est un copolymère d'éther méthylvinylique et d'un maléate de monoalkyle.

3. Vernis à ongles selon la revendication 1 ou 2, caractérisé en ce que l'agent feuillogène insoluble dans l'eau est un copolymère d'éther méthylvinylique et de maléate de mono-n-butyle.

4. Vernis à ongles selon une ou plusieurs des revendications 1 à 3, caractérisé en ce que la substance à action antimycosique est le tioconazole et/ou l'éconazole.

5. Vernis à ongles selon une ou plusieurs des revendications 1 à 4, caractérisé en ce que la substance active est contenue en une quantité d'environ 2 à 80 % en poids, par rapport à la quantité des composants non volatils.

6. Vernis à ongles selon une ou plusieurs des revendications 1 à 5, caractérisé en ce que la substance active est contenue en une quantité d'environ 10 à 60 % en poids, par rapport à la quantité des composants non volatils.

7. Vernis à ongles selon une ou plusieurs des revendications 1 à 6, caractérisé en ce qu'il contient la substance active en une quantité d'environ 0,5 à 20 % en poids, par rapport à la quantité des composants volatils et des composants non volatils.

8. Vernis à ongles selon une ou plusieurs des revendications 1 à 7, caractérisé en ce qu'il contient la substance active en une quantité d'environ 2 à 15 % en poids par rapport à la quantité des composants volatils et des composants non volatils.

9. Procédé pour la préparation d'un vernis à ongles ayant une teneur en au moins un agent feuillogène insoluble dans l'eau et en au moins une substance à action antimycosique, caractérisé en ce qu'on mélange au moins un agent feuillogène insoluble dans l'eau, du type défini dans la revendication 1,

avec au moins une substance à action antimycosique qui est également définie dans la revendication 1, ainsi qu'éventuellement avec d'autres composants utilisés habituellement pour la préparation de vernis à ongles.

**10.** Utilisation d'une ou plusieurs des substances à action antimycosique, définies dans la revendication 1, en tant qu'additifs dans un(des) vernis à ongles comportant au moins un agent feuillogène insoluble dans l'eau.